# EUROPEAN PATENT SPECIFICATION

(11) **EP 2 244 751 B1**
(45) Date of publication and mention of the grant of the patent: **18.09.2013**
(21) Application number: 09700303.2
(22) Date of filing: 09.01.2009
(51) Int. Cl.: A61L 27/36, A61L 24/02, A61L 27/12, A61F 2/28

(54) **A METHOD FOR PRODUCING A BONE REPAIR COMPOSITION**
VERFAHREN ZUR HERSTELLUNG VON KNOCHENREPARATURZUSAMMENSETZUNG
PROCÉDÉ DE PRODUCTION D'UNE COMPOSITION DE RÉPARATION OSSEUSE

(30) Priority: 09.01.2008 GB 0800371; 09.01.2008 GB 0800370
(43) Date of publication of application: 03.11.2010
(73) Proprietor: Oswestry Tissue Bank Ltd., Wrexham LL14 5NS (GB)
(72) Inventor: MYINT, Peter, Oswestry SY10 7AG (GB); KUIPER, Jan Herman, Oswestry Shropshire SY112RU (GB)
(74) Representative: Ablett, Graham Keith
(86) International application number: PCT/GB2009/050017
(87) International publication number: WO 2009/087421

(56) References cited:
- WO-A1-98/42280
- WO-A1-2004/060430
- WO-A2-2004/091435
- US-A- 6 053 970
- US-B1- 6 214 368

## Description

The present invention concerns a method for producing a bone repair composition, in particular, a bone repair composition for use in impaction grafting, for example in revision total joint replacement surgery.

In this connection, total joint replacement surgery, in particular for the hip or knee, is relatively successful. Nevertheless, over time, joint prosthesis failure can occur, necessitating revision surgery. The most common reason for prosthesis failure is aseptic loosening, where, for various reasons, bone around the prosthesis is progressively resorbed until the prosthesis has lost its fixation.

During joint replacement surgery, coping with the bone loss caused by aseptic loosening is extremely challenging. Some revision techniques involve using larger prostheses and/or more bone cement to fill the spaces left by resorbed bone. However, such techniques do not attempt to counteract the loss of bone tissue. As such, yet further revision surgeries will result in a vicious cycle of ever reducing bone mass and, consequently, failure rates are much higher than for initial replacement operations. This is a particular problem in revision hip replacement surgery, as the femoral stem of the prosthesis must be inserted into the medullary canal of the femur and be supported by the surrounding bone mass.

To overcome the above issue, techniques have been developed to try to replace lost bone before implanting a new replacement joint prostheses. Impaction bone grafting using morselized bone is one such method that has been used for revision hip replacement surgery. In this method, morselized allograft bone granules, typically 1-5mm in diameter, are packed into the medullary canal. A cannulated tamp is positioned during the packing process and, once removed, forms a neo-medullary canal. A revision femoral stem prosthesis is then implanted into the neo-medullary canal using PolyMethylMethAcrylate bone cement. Tight packing of the bone chips promotes stability of the revision prosthesis, and spaces between bone chips allow ingrowth of blood vessels and invasion by bone cells, promoting replacement of the bone graft by new viable bone. These spaces also allow penetration of PMMA bone cement. There is therefore a balance between the mechanical demands of enabling initial stability of the prosthesis and achieving a consistency suitable for long term enhancement of bone development.

Whilst the above technique has been successful, it involves the use of morselized allograft bone. This is often prepared in the operating theatre by grinding femoral heads. However, the continued use of allograft bone is of increasing concern because of high costs, limited supply and the risk of disease transmission.

To address these issues, morselized allograft bone has been mixed with synthetic bone substitutes, such as calcium phosphate granules, sized to match the morselized allograft bone, in order to reduce the amount of allograft used. However, the synthetic bone substitutes have different mechanical and handling properties compared to allograft bone, so, though they can mitigate the problems of expense, supply and disease transmission, surgeons are often reluctant to use them in practice. Furthermore, recommended practice is to mix bone substitutes with allograft bone, usually in a 50:50 ratio, and therefore the problems with the allograft material are not wholly avoided.

A particular problem with known bone substitutes is that during the impaction procedure outlined above, a large number of the synthetic bone substitute particles are displaced and fall down the narrow neo-medullary canal each time the cannulated tamp is withdrawn. This perturbs or unsettles the neo-medullary canal and compromises its interface with the prosthesis femoral stem. It has been suggested that this is because the synthetic bone substitute particles are less "sticky" or "cohesive" than allograft bone. To improve the cohesiveness of the materials, some surgeons add clotted blood to the mixture of morselized allograft bone and synthetic bone substitute. Although this offers some improvement, it still fails to produce a mixture as cohesive as pure morselized allograft bone, which remains the preferred material for this procedure.

WO 2004/091435 discloses osteogenic bone implant compositions that approximate the chemical composition of natural bone. The organic component of these implant compositions is osteoinductive despite the presence of the inorganic component and, further, is present in an amount sufficient to maximize the implant's regenerative capabilities without compromising its formability and mechanical strength.

US 6,214,368 discloses a process for converting a standard inert amorphous calcium phosphate precipitate into highly reactive amorphous solids. The amorphous solids can be used to react with other calcium phosphate solids to form a poorly-crystalline synthetic hydroxyapatite that provides both bioactivity and structural integrity. This amorphous material can be reacted with other calcium phosphates at or below 37°C to form a bone-like material consisting of poorly crystalline hydroxyapatite.

US 6,053,970 discloses storage stable, two component apatitic cement compositions and methods for their production. The dry component of the cement compositions comprise basic calcium source particles partially coated with a partially neutralized acidic calcium phosphate, the dry component of the compositions may be produced through mechanical mixing of an acidic phosphate source with a basic calcium particle source in the presence of a liquid phase, during which time reaction occurs between the two sources. Neutralization of the acidic phosphate source is stopped prior to completion through removal of available water. The resultant basic calcium source particles partially coated with a partially neutralized acidic calcium phosphate are storage stable and, when combined with a lubricant, form a rapidly setting apatitic product.

WO 2004/060430 discloses end-capped polymers, methods for making those end-capped polymers, compositions containing those end-capped polymers and methods for using those compositions. One of the disclosed end-capped copolymers may be combined with natural or synthetic bone minerals, bone tissue, collagen tissue, bone protein or combinations or derivatives of those materials to form a tissue repair composition.

WO 98/42280 discloses a cement delivery system with a container for holding cement. A syringe is provided in the container to force the cement from the container into a tube. The tube, which is locatable within a bone canal, has a central locating means for locating the tube over a guide rod. The locating means of the tube is in the form of a number of projections from an internal surface of the tube.

The present invention seeks to overcome the above problems associated with the prior art.

According to an aspect of the present invention there is provided a method for producing a bone repair composition comprising steps of: mixing a first aqueous calcium phosphate suspension with bone graft granules to form an intermediate mixture; and mixing the intermediate mixture with a second aqueous calcium phosphate suspension; wherein said first and second aqueous calcium phosphate suspensions comprise crystalline calcium phosphate nano-particles; and wherein said first aqueous calcium phosphate suspension contains a lower weight concentration of calcium phosphate than the second aqueous calcium phosphate suspension.

In this way, the calcium phosphate suspension forms a paste like binder for the bone graft granules, thereby enhancing cohesion between the bone graft granules. In particular, during the mixing process, the first, lower concentration, aqueous calcium phosphate suspension coats the bone graft granules. It is believed that this prevents excessive dehydration during the subsequent mixing step. Following this, the more concentrated second aqueous calcium phosphate suspension is mixed in. The resultant composition exhibits excellent clinical handling properties and cohesiveness. These improvements in cohesiveness allow the use of synthetic bone substitute graft granules, whilst addressing the previous issue of synthetic granules falling down the narrow neo-medullary canal. Moreover, as the calcium phosphate paste promotes cell proliferation of the bone formation cells, its presence in the composition as whole helps to promote ingrowth of blood vessels and invasion by bone cells, leading to the replacement of the composition by new viable bone.

Conveniently, the bone graft granules are a synthetic bone substitute. Due to the greatly enhanced cohesiveness provided by the calcium phosphate paste binder, the composition can use predominantly or entirely synthetic bone substitute materials. This thereby avoids the problems of disease transmission and high cost associated with allograft bone materials, without compromising clinical handling.

Preferably, the bone graft granules comprise hydroxyapatite (HAP). Hydroxyapatite has a high hardness and toughness, making it particularly suitable for impaction grafting techniques, where tight packing is desired. The bone graft granules may also comprise tricalcium phosphate (TCP). The bone graft granules may also comprise autograft, allograft, or xenograft bone.

In one embodiment the bone graft granules comprise demineralised bone matrix (DBM).

Preferably, the first aqueous calcium phosphate suspension is mixed with the bone graft granules to give a composition of ratio 30-50 : 50-70 first aqueous calcium phosphate suspension to bone graft granules by weight. In a preferred embodiment the first aqueous calcium phosphate suspension is mixed with the bone graft granules to give a composition of ratio 30:50 first aqueous calcium phosphate suspension to bone graft granules by weight. In an alternative embodiment, the first aqueous calcium phosphate suspension is mixed with the bone graft granules to give a composition of ratio 40:60 first aqueous calcium phosphate suspension to bone graft granules by weight. It has been found that these quantities allow the first aqueous calcium phosphate suspension to particularly effectively coat the bone graft granules during the first mixing step, resulting in a final composition having improved handling properties.

Preferably, the second aqueous calcium phosphate suspension is mixed with the intermediate mixture at a composition of ratio 20-40 : 60-80 second aqueous calcium phosphate suspension to bone graft granules by weight. More preferably, the second aqueous calcium phosphate suspension is mixed with the intermediate mixture at a composition of ratio 30 : 70 second aqueous calcium phosphate suspension to bone graft granules by weight. It has been found that these quantities result in a final composition having particularly improved handling properties and cohesiveness.

Conveniently, calcium phosphate is present at a concentration of 5 wt% to 20 wt% in said first aqueous calcium phosphate suspension. Preferably, calcium phosphate is present at a concentration of 12 wt% to 18 wt% in said first aqueous calcium phosphate suspension. It has been found that these concentrations are particularly effective at coating the bone graft granules during the first mixing step, resulting in a final composition having improved handling properties.

Conveniently, calcium phosphate is present at a concentration of 20 wt% to 40 wt% in said second aqueous calcium phosphate suspension. Preferably, calcium phosphate is present at a concentration of 20 wt% to 30 wt% in said second aqueous calcium phosphate suspension. It has been found that these concentrations result in a final composition having particularly improved handling properties and cohesiveness.

Preferably, the crystalline calcium phosphate nano-particles are fully crystalline.

Optionally, the composition may further comprise growth factors and/or therapeutic agents. In this way, the resultant composition can be provided with additional components, depending on its application, to further improve clinical results.

Conveniently, the composition comprises more than 35 wt% water.

Illustrative examples of the present invention will now be described below in detail.

In this connection, a method of preparing a bone grafting composition according to an embodiment of the present invention will now be described.

Firstly, an aqueous stock solution (suspension) of ∼8% w/w of calcium phosphate nano particles is heated to dry it. As it dries, the relative concentration of calcium phosphate increases until two calcium phosphate pastes are formed, the first paste having a concentration of 13-17% w/w and the second having a concentration of 20-30% w/w. In this specific embodiment, the pastes have a concentration of approximately 14% w/w for the first paste, and 26% w/w for the second paste. The concentration is measured by weighing an oven-dried sample of the mixture until a constant weight is reached. In an alternative embodiment, rather than heat drying, "vacuum filtration" could be used to obtain the desired paste concentrations.

In this connection, the aqueous suspension of calcium phosphate nano particles contains fully crystalline calcium phosphate phases, such as hydroxyapatite, tri-calcium phosphate, or tri-calcium orthophosphate. This crystalline structure means that the calcium phosphate does not self-harden in the presence of water and, hence, the suspension remains as a paste or putty, rather than forming a hardened solid. In this embodiment, the aqueous stock suspension is hydroxyapatite nano-paste. As such, the pure hydroxyapatite has a hexagonal crystal structure and an acicular habit of nanometer sized crystals forming clusters, i.e. needle shaped crystals. The chemical formula for this is Ca₁₀(PO₄)₆(OH)₂ and the Ca:P ratio is 1.67.

As bone graft granules, hydroxyapatite granules of 2-4mm particle size are weighed and thoroughly mixed with an amount of the first calcium phosphate paste (∼14 wt%) to give a composition of ratio 40:60 first calcium phosphate paste to hydroxyapatite by weight.

Once the above intermediate mixture is fully mixed, an amount of second calcium phosphate paste (∼26 wt%) is then added to the intermediate mixture to obtain a ratio of 30:70 second calcium phosphate paste to hydroxyapatite by weight. This is then thoroughly mixed to produce the final composition.

Mechanical testing of the cohesion of the final composition will now be described. The composition was placed in a cylinder mould with an internal diameter of 17mm diameter and a height of 40mm. A 1 kg weight, comparable to an operative hammer, was dropped 20 times from a height of 50mm on a piston to compact the composition. The mould was split lengthwise to carefully remove the impacted sample. The height of all the samples was measured after impaction.

The cylindrical samples were transferred to a 5 KN servo-hydraulic testing machine (manufactured by ESH Testing Ltd, Brierley Hill, UK). The specimens were loaded at a strain rate of 2.5% of the initial sample height per minute, to a maximum of 15% of sample height or until failure was achieved. Stress-strain diagrams were then compiled from the results and from these, the compressive strength at failure or at 15% strain was determined. The sample size, loading rate and definition of failure were chosen according to an international standard. The cohesion or shear strength at zero total normal stress for each sample was then calculated as half the compressive strength. The above procedure was repeated three times to give an average cohesion value. All statistical analyses were performed using Systat 11 (Systat Software Inc., Richmond, California). The cohesion values from three experiments were 20, 20 and 25, giving a mean value of 21.7 kPa.

The above recorded cohesion values for the present invention are comparative to comparative samples formed of allograft and clotted blood. In contrast, however, comparative samples of allograft without clotted blood, allograft and synthetic mixtures, and synthetics achieve much lower cohesion values, as shown in Table 1 below.

**TABLE 1 : Mean cohesion values for each separate experimental group. From Oakley J & Kuiper JH. JBJS Vol 88B (No 6) June 2006, 828-831.**

| **Bone (%)** | **Extender type** | **Clotted blood added** | **Cohesion (KPa)** |
|---|---|---|---|
| 100 | - | No | 11.9 |
| 100 | - | Yes | 23.7 |
| 50 | HAP | No | 0.9 |
| 50 | HAP | Yes | 0.5 |
| 50 | HAP/TCP | No | 3.5 |
| 50 | HAP/TCP | Yes | 10.6 |
| 0 | HAP | No | 0 |
| 0 | HAP/TCP | Yes | 0 |

Accordingly, with the present invention, the calcium phosphate paste enables particulate bone grafts, such as synthetic bone substitute granules, to be used in impaction bone grafting where cohesion between the particles is required.

It has been particularly found that the two step mixing process employed in the present invention achieves pronounced improvements in cohesion between the bone graft granules. In contrast, comparative examples using a single step mixing procedure exhibited much lower cohesion. It is believed this is because the first mixing step, using a lower calcium phosphate paste concentration, provides a thin coating over the granules which prevents excessive dehydration when the second paste is added.

Furthermore, as discussed above, with the present invention, the nano particles of calcium phosphate are crystalline. As such, the composition remains paste-like and fluid once mixed. This allows the calcium phosphate nano particles and the bone graft granules disbursed therein to remain mobile within the resultant composition, permitting movement thereof as well as bone ingrowth. This avoids limiting the expression of the components' osteoinductive function. As a result, the composition can achieve high levels of osteoinduction.

Furthermore, in the application of impaction grafting, a further important property of the composition, along with cohesion, is the ability for bone cement to penetrate into the bone repair composition. This can be measured in mm and affects the stability of an implant after implantation. That is, before bone ingrowth occurs, the bone cement used to stabilise the joint between the implant and the newly impacted bone grafts. If bone cement is unable to penetrate into the bone repair composition, effective bonding between the implant and the bone will not occur. Conversely, if the penetration of the bone cement is too high, the bone repair composition may be unable to work effectively to promote bone ingrowth. Accordingly, for impaction grafting, it is preferable that the bone repair composition has a penetration values of between approximately 1mm-2mm, along with cohesion value of 5-25 KPa.

As an illustration, the following results were achieved with specific examples of the present invention.

**TABLE 2 : Cohesion and Penetration values for the application of impaction grafting.**

| **First Aqueous Calcium Phosphate Suspension** | | **Second Aqueous Calcium Phosphate Suspension** | | **Cohesion (KPa)** | **Penetration (mm)** |
|---|---|---|---|---|---|
| **Concentration (% w/w)** | **Mix Ratio with Hydroxyapatite granules** | **Concentration (% w/w)** | **Mix Ratio with Hydroxyapatite granules** | | |
| 13% | 30:70 | 30% | 30:70 | 10 | 2 |
| 13% | 50:50 | 25% | 30:70 | 10 | 1.5 |
| 15% | 30:70 | 25% | 30:70 | 8 | 2 |
| 15% | 30:70 | 30% | 30:70 | 20 | 1 |
| 15% | 50:50 | 20% | 30:70 | 12 | 1.25 |
| 17% | 50:50 | 25% | 30:70 | 10 | 1.5 |

Although the present invention has been described in the above illustrated embodiment, the present invention is not limited solely to this particular embodiment.

For example, in the above embodiment, hydroxyapatite has been used as the bone graft granules, although it will be understood that other materials could also be used, or mixtures of granules could be used. For example, materials such as tricalcium phosphate granules, other synthetic bone substitutes, or harvested bone such as autograft, allograft, or xenograft bone.

Demineralised bone matrix (hereinafter DBM) could also be used as the bone graft granules. In this connection, DBM is typically provided in the form of a fine powder, with particle sizes of 74-420 µm. However, as a consequence of this, DBM can be extremely difficult to handle in clinical applications. The present invention allows the calcum phosphate to be used as a carrier to enhance cohesion between the DBM particles and thereby provide better handling properties of the DBM.

In this connection, for example, a first aqueous crystalline calcium phosphate suspension of 14% w/w is firstly mixed with DBM to form an intermediate mixture. After this, a second aqueous crystalline calcium phosphate suspension of 25% w/w is mixed into the intermediate mixture. Preferably, the first and second aqueous calcium phosphate components are mixed with the DBM to give a composition ratio of 40-60:20-40:10-30, by weight, first aqueous crystalline calcium phosphate component to second aqueous crystalline calcium phosphate component to the DBM, respectively. In a particularly preferred embodiment the first and second aqueous crystalline calcium phosphate components are mixed with the DBM to give a composition ratio of 50:30:20, by weight, first aqueous crystalline calcium phosphate component to second aqueous crystalline calcium phosphate component to the DBM, respectively.

Furthermore, different bone graft granule sizes could be used to optimise handling properties and characteristics depending on surgeon preference or the particular clinical application. Similarly, the concentrations and quantities of the first and second calcium phosphate suspensions can be varied to alter the properties of the final composition.

Moreover, it will be understood that further mixing steps could be introduced, for example to introduce additional agents such as growth factors and therapeutic agents.

Finally, although the above example describes the manufacture of a bone treatment composition for impaction bone grafting in joint replacement surgery, it will also be understood that the present invention could be used for other bone repair applications, for example to repair bone cysts or bone voids.

## Claims

1. A method for producing a bone repair composition comprising steps of:
mixing a first aqueous calcium phosphate suspension with bone graft granules to form an intermediate mixture; and
mixing the intermediate mixture with a second aqueous calcium phosphate suspension,
wherein said first and second aqueous calcium phosphate suspensions comprise crystalline calcium phosphate nano-particles, and
wherein said first aqueous calcium phosphate suspension contains a lower weight concentration of calcium phosphate than the second aqueous calcium phosphate suspension.

2. A method according to claim 1 wherein the bone graft granules comprise at least one selected from the group of:
a synthetic bone substitute, hydroxyapatite (HAP), tricalcium phosphate (TCP), autograft, allograft, and xenograft bone.

3. A method according to claim 1 wherein the bone graft granules comprise demineralised bone matrix (DBM).

4. A method according to any preceding claim wherein the first aqueous calcium phosphate suspension is mixed with the bone graft granules to give a composition of ratio 30-50 : 50-70 first aqueous calcium phosphate suspension to bone graft granules by weight.

5. A method according to any preceding claim wherein the first aqueous calcium phosphate suspension is mixed with the bone graft granules to give a composition of ratio 30:50 first aqueous calcium phosphate suspension to bone graft granules by weight.

6. A method according to any preceding claim wherein the first aqueous calcium phosphate suspension is mixed with the bone graft granules to give a composition of ratio 40:60 first aqueous calcium phosphate suspension to bone graft granules by weight.

7. A method according to any preceding claim wherein the second aqueous calcium phosphate suspension is mixed with the intermediate mixture at a composition of ratio 20-40 : 60-80 second aqueous calcium phosphate suspension to bone graft granules by weight.

8. A method according to any preceding claim wherein the second aqueous calcium phosphate suspension is mixed with the intermediate mixture at a composition of ratio 30 : 70 second aqueous calcium phosphate suspension to bone graft granules by weight.

9. A method according to any preceding claim wherein calcium phosphate is present at a concentration of 5 wt% to 20 wt% in said first aqueous calcium phosphate suspension.

10. A method according to any preceding claim wherein calcium phosphate is present at a concentration of 12 wt% to 18 wt% in said first aqueous calcium phosphate suspension.

11. A method according to any preceding claim wherein calcium phosphate is present at a concentration of 20 wt% to 40 wt% in said second aqueous calcium phosphate suspension.

12. A method according to any preceding claim wherein calcium phosphate is present at a concentration of 20 wt% to 30 wt% in said second aqueous calcium phosphate suspension.

13. A method according to any preceding claim, wherein the crystalline calcium phosphate nano-particles are fully crystalline.

14. A method according to any preceding claim further comprising the step of mixing in growth factors and/or therapeutic agents.

15. A method according to any preceding claim wherein the resultant composition comprises more than 35 wt% water.

## Patentansprüche

1. Ein Verfahren zur Herstellung einer Knochenreparatur-Zusammensetzung, das folgende Schritte umfasst:
Mischen einer ersten wässrigen Calciumphosphat-Suspension mit Knochentransplantatgranulat, um ein Zwischengemisch zu bilden; und
Mischen des Zwischengemisches mit einer zweiten wässrigen Calciumphosphat-Suspension,
wobei die erste und die zweite wässrige Calciumphosphat-Suspension kristalline Calciumphosphat-Nanopartikel enthalten, und
wobei die erste wässrige Calciumphosphat-Suspension eine geringere Gewichtskonzentration von Calciumphosphat enthält als die zweite wässrige Calciumphosphat-Suspension.

2. Ein Verfahren gemäß Anspruch 1, wobei das Knochentransplantat-Granulat mindestens eines aus der folgenden Gruppe beinhaltet: ein synthetisches Knochenersatzmaterial, Hydroxylapatit (HAP), Tricalciumphosphat (TCP), Autotransplantat-, Allotransplantat- und Xenotransplantat-Knochen.

3. Ein Verfahren gemäß Anspruch 1, wobei das Knochentransplantatgranulat demineralisierte Knochenmatrix (DBM) umfasst.

4. Ein Verfahren gemäß einem vorhergehenden Anspruch, wobei die erste wässrige Calciumphosphat-Suspension mit dem Knochentransplantatgranulat vermischt wird, um eine Zusammensetzung im Verhältnis von 30-50 : 50-70 der ersten wässrigen Calciumphosphat-Suspension zum Knochentransplantatgranulat nach Gewicht zu erhalten.

5. Ein Verfahren gemäß einem vorhergehenden Anspruch, wobei die erste wässrige Calciumphosphat-Suspension mit dem Knochentransplantatgranulat vermischt wird, um eine Zusammensetzung im Verhältnis von 30:50 der ersten wässrigen Calciumphosphat-Suspension zum Knochentransplantatgranulat nach Gewicht zu erhalten.

6. Ein Verfahren gemäß einem vorhergehenden Anspruch, wobei die erste wässrige Calciumphosphat-Suspension mit dem Knochentransplantatgranulat vermischt wird, um eine Zusammensetzung im Verhältnis von 40:60 der ersten wässrigen Calciumphosphat-Suspension zum Knochentransplantatgranulat nach Gewicht zu erhalten.

7. Ein Verfahren gemäß einem vorhergehenden Anspruch, wobei die zweite wässrige Calciumphosphat-Suspension mit dem Zwischengemisch in einer Zusammensetzung im Verhältnis von 20-40 : 60-80 der zweiten wässrigen Calciumphosphat-Suspension zum Knochentransplantatgranulat nach Gewicht vermischt wird.

8. Ein Verfahren gemäß einem vorhergehenden Anspruch, wobei die zweite wässrige Calciumphosphat-Suspension mit dem Zwischengemisch in einer Zusammensetzung im Verhältnis von 30:70 der zweiten wässrigen Calciumphosphat-Suspension zum Knochentransplantatgranulat nach Gewicht verwischt wird.

9. Ein Verfahren gemäß einem vorhergehenden Anspruch, wobei Calciumphosphat in einer Konzentration von 5 Gew.-% bis 20 Gew.-% in der ersten wässrigen Calciumphosphat-Suspension vorhanden ist.

10. Ein Verfahren gemäß einem vorhergehenden Anspruch, wobei Calciumphosphat in einer Konzentration von 12 Gew.-% bis 18 Gew.-% in der ersten wässrigen Calciumphosphat-Suspension vorhanden ist.

11. Ein Verfahren gemäß einem vorhergehenden Anspruch, wobei Calciumphosphat in einer Konzentration von 20 Gew.-% bis 40 Gew.-% in der zweiten wässrigen Calciumphosphat-Suspension vorhanden ist.

12. Ein Verfahren gemäß einem vorhergehenden Anspruch, wobei Calciumphosphat in einer Konzentration von 20 Gew.-% bis 30 Gew.-% in der zweiten wässrigen Calciumphosphat-Suspension vorhanden ist.

13. Ein Verfahren gemäß einem vorhergehenden Anspruch, wobei die kristallinen Calciumphosphat-Nanopartikel vollkommen kristallin sind.

14. Ein Verfahren gemäß einem vorhergehenden Anspruch, das überdies den Vorgang des Mischens in Wachstumsfaktoren und/oder therapeutischen Wirkstoffen umfasst.

15. Ein Verfahren gemäß eine vorhergehenden Anspruch, worin die entstandene Zusammensetzung mehr als 35 Gew.-% Wasser enthält.

## Revendications

1. Un procédé de production d'une composition de réparation osseuse comprenant les opérations suivantes :
le mélange d'une première suspension de phosphate de calcium aqueuse avec des granules de greffe osseuse de façon à former un mélange intermédiaire, et
le mélange du mélange intermédiaire avec une deuxième suspension de phosphate de calcium aqueuse,
où lesdites première et deuxième suspensions de phosphate de calcium aqueuses contiennent des nanoparticules de phosphate de calcium cristallin, et
où ladite première suspension de phosphate de calcium aqueuse contient une concentration en poids plus faible de phosphate de calcium que la deuxième suspension de phosphate de calcium aqueuse.

2. Un procédé selon la Revendication 1 où les granules de greffe osseuse contiennent au moins un élément sélectionné dans le groupe composé de : un substitut osseux synthétique, hydroxyapatite (HAP), triphosphate de calcium (TCP), os d'autogreffe, d'allogreffe et de xénogreffe.

3. Un procédé selon la Revendication 1 où les granules de greffe osseuse contiennent une matrice osseuse déminéralisée (DBM).

4. Un procédé selon l'une quelconque des Revendications précédentes où la première suspension de phosphate de calcium aqueuse est mélangée avec les granules de greffe osseuse de façon à donner une composition d'un rapport 30-50 : 50-70 première suspension de phosphate de calcium aqueuse sur granules de greffe osseuse en poids.

5. Un procédé selon l'une quelconque des Revendications précédentes où la première suspension de phosphate de calcium aqueuse est mélangée avec les granules de greffe osseuse de façon à donner une composition d'un rapport 30:50 première suspension de phosphate de calcium aqueuse sur granules de greffe osseuse en poids.

6. Un procédé selon l'une quelconque des Revendications précédentes où la première suspension de phosphate de calcium aqueuse est mélangée avec les granules de greffe osseuse de façon à donner une composition d'un rapport 40:60 première suspension de phosphate de calcium aqueuse sur granules de greffe osseuse en poids.

7. Un procédé selon l'une quelconque des Revendications précédentes où la deuxième suspension de phosphate de calcium aqueuse est mélangée avec le mélange intermédiaire à une composition d'un rapport de 20-40 : 60-80 deuxième suspension de phosphate de calcium aqueuse sur granules de greffe osseuse en poids.

8. Un procédé selon l'une quelconque des Revendications précédentes où la deuxième suspension de phosphate de calcium aqueuse est mélangée avec le mélange intermédiaire à une composition d'un rapport 30 : 70 deuxième suspension de phosphate de calcium aqueuse sur granules de greffe osseuse en poids.

9. Un procédé selon l'une quelconque des Revendications précédentes où le phosphate de calcium est présent à une concentration de 5% en poids à 20% en poids dans ladite première suspension de phosphate de calcium aqueuse.

10. Un procédé selon l'une quelconque des Revendications précédentes où le phosphate de calcium est présent à une concentration de 12% en poids à 18% en poids dans ladite première suspension de phosphate de calcium aqueuse.

11. Un procédé selon l'une quelconque des Revendications précédentes où le phosphate de calcium est présent à une concentration de 20% en poids à 40% en poids dans ladite deuxième suspension de phosphate de calcium aqueuse.

12. Un procédé selon l'une quelconque des Revendications précédentes où le phosphate de calcium est présent à une concentration de 20% en poids à 30% en poids dans ladite deuxième suspension de phosphate de calcium aqueuse.

13. Un procédé selon l'une quelconque des Revendications précédentes, où les nanoparticules de phosphate de calcium cristallin sont totalement cristallines.

14. Un procédé selon l'une quelconque des Revendications précédentes comprenant en outre l'opération de mélange dans des facteurs de croissance et/ou des agents thérapeutiques.

15. Un procédé selon l'une quelconque des Revendications précédentes où la composition résultante contient plus de 35% en poids d'eau.
